# DEMANDE DE BREVET EUROPEEN

(11) **EP 0 972 509 A1**
(43) Date de publication de la demande: **19.01.2000**
(21) Numéro de dépôt: 99401610.3
(22) Date de dépôt: 28.06.1999
(51) Int. Cl.: A61K 7/02, A61K 7/035, A61K 7/38

(54) **Composition cosmétique comprenant des pigments et un agent anti-transpirant, utilisation d'une telle composition**

(30) Priorité: 17.07.1998 FR 9809155
(71) Demandeur: L'OREAL, 75008 Paris (FR)
(72) Inventeur: Bazin, Roland, 91570 Bievres (FR); Bara, Isabelle, 75013 Paris (FR)
(74) Mandataire: Brédeville, Odile Marie

(57) **Abrégé**

La présente invention est relative à une composition notamment cosmétique, pouvant notamment se présenter sous la forme d'une émulsion, d'un gel, d'une dispersion vésiculaire, ladite composition comprenant une phase pulvérulente à une teneur inférieure ou égale à 80% en poids, par rapport au poids total de la composition, cette composition comprenant au moins un pigment et au moins un agent antitranspirant.

L'invention concerne également l'utilisation d'une telle composition en cosmétique en particulier pour le maquillage afin d'améliorer la tenue de la couleur de la composition.

## Description

La présente invention a pour objet une composition cosmétique comprenant un agent antitranspirant, cette composition pouvant en particulier se présenter sous la forme d'une émulsion, d'un gel, d'une dispersion vésiculaire, et susceptible d'être utilisée pour le maquillage de la peau, des semi-muqueuses, des muqueuses et/ou des phanères.

Les compositions cosmétiques, notamment de maquillage telles que les rouges à lèvres, les anticernes ou les fonds de teint, comprennent généralement des corps gras tels que des huiles et des cires, et une phase particulaire généralement composée de charges et de pigments. Elles peuvent ainsi se présenter, par exemple dans les cas des rouges à lèvres, sous forme de stick ou bâton ou sous forme de pâte souple. Elles sont alors souvent sous la forme d'une composition anhydre. Les compositions de maquillage peuvent également comprendre de l'eau ou une phase hydrophile, et se présenter alors notamment sous forme d'émulsion huile-dans-eau, eau-dans-huile, émulsion multiple, solution ou gel aqueux, notamment lorsqu'il s'agit d'un fond de teint, de crème teintée, de crème de soin ou d'un produit solaire.

Ces compositions, qui sont avant tout des produits de maquillage, ont pour vocation première d'embellir la personne humaine en soulignant par exemple les qualités esthétiques et/ou encore en dissimulant les imperfections du visage.

Selon le maquillage que l'on désire obtenir, léger, discret ou au contraire accentué, on apporte au visage tantôt une nuance proche de la carnation naturelle de la personne, tantôt une couleur plus soutenue qui rehausse le teint naturel : on utilise pour cela les fonds de teint. On peut également accentuer certains reliefs en apportant des tons plus soutenus en utilisant les blushes par exemple. Pour rehausser le regard, on utilise des teintes plus tranchantes couvrant toutes les nuances des couleurs. Enfin, on met les lèvres en valeur en leur apportant des couleurs généralement foncées.

Dans tous les cas, le résultat est obtenu grâce à la présence de pigments et/ou colorants dans ces différentes compositions dont chacune a une fonction bien particulière. Ainsi, la nature des pigments, leur couleur mais surtout leur proportion au sein de chacune de ces compositions sont primordiales pour obtenir la nuance souhaitée et ainsi l'effet désiré.

Il est donc également très important que la répartition de ces pigments et/ou colorants reste constante et homogène au cours du temps, c'est-à-dire même plusieurs heures après l'application. Afin de conserver au visage un maquillage d'ensemble esthétique, il est primordial que les couleurs conférées par chacune des compositions (fond de teint, blush, fard à paupière, rouge à lèvres, etc...) au moment de leur application ne se mélangent pas.

Or, il s'avère bien souvent que l'ensemble du maquillage se détériore peu après l'application des produits : on a en effet constaté que certaines compositions avaient tendance à se propager à l'intérieur des ridules et/ou des rides de la peau, dans le cas des fonds de teint ; dans les ridules qui entourent les lèvres, dans le cas des rouges à lèvres ; dans les plis de la paupière, dans le cas des fards à paupières. On a également constaté, dans le cas notamment des fards à paupières, l'apparition de stries dans le maquillage, générées par les mouvements des paupières. On peut également constater l'apparition de parties luisantes ou brillantes ou encore la dilution de la couvrance et de la couleur. Cela peut être dû à de multiples facteurs comme par exemple la chaleur, l'humidité. En particulier, la répartition des couleurs n'est plus aussi homogène. On peut ainsi voir apparaître sur le visage maquillé des parties brillantes et des parties assombries qui vont à l'encontre de l'effet esthétique recherché.

Or les femmes d'aujourd'hui désirent des produits de maquillage qui leur donnent un teint frais et une bonne mine tout au long de la journée tout en étant pratiques d'utilisation. Ainsi, elles ne désirent pas réappliquer leurs produits plusieurs fois par jour dans le but d'avoir un maquillage net de façon permanente.

Il serait également intéressant de disposer d'un seul produit qui pourrait être utilisé en été, en hiver, dans des pays froids ainsi que dans des pays chauds. Un tel produit universel serait très pratique pour les femmes qui voyagent, il permettrait de gagner du temps et serait de moindre coût.

Il subsiste donc le besoin d'une composition cosmétique, notamment de maquillage, qui non seulement possède de bonnes propriétés cosmétiques mais également qui confère à la peau une coloration dont la répartition reste homogène et constante au cours du temps et après application sur la peau, en particulier sur le visage.

Or, la Demanderesse a découvert de façon surprenante qu'en introduisant dans des compositions comprenant des pigments un agent antitranspirant, il était possible de réaliser des compositions de maquillage dont la répartition des couleurs est constante au cours du temps, en particulier après l'application sur la peau humaine.

L'invention a donc pour objet une composition cosmétique, notamment de maquillage, comprenant une phase pulvérulente à une teneur inférieure ou égale à 80 % en poids, par rapport au poids total de la composition, cette phase pulvérulente comprenant au moins un pigment, caractérisée en ce qu'elle comprend en outre au moins un agent antitranspirant.

Dans l'article "Preventing Makeup Darkening During Usage", Akira Matsueda and Tsuyoshi Ogihara, Cosmetics & Toiletries, Vol. 111, November 1996, il est enseigné que la sécrétion de sébum provoque l'assombrissement des produits de maquillage. Pour remédier à cet inconvénient, il est proposé d'introduire dans les compositions une silice poreuse comprenant un pigment blanc.

Toutefois, il n'a jamais été proposé d'introduire dans un produit de maquillage, en particulier pour le visage, un agent antitranspirant, notamment dans le but d'améliorer la tenue de la couleur après application au cours du temps et de diminuer la brillance du maquillage.

Les compositions selon l'invention confèrent au visage une coloration particulièrement stable et dont la répartition reste homogène au cours du temps, même plusieurs heures après l'application.

Ces compositions présentent de plus une excellente tenue : elles ne migrent pas dans les plis tels que les paupières ou les rides, on n'observe pas d'accumulation de produit dans certaines parties du visage. Elles transfèrent également très peu sur les supports tels que les tasses ou les vêtements. Elles confèrent à la peau un maquillage homogène, une bonne matité. Elles évitent au visage un aspect poisseux et maintiennent sur ce dernier un état plutôt sec particulièrement recherché dans des climats chauds et humides.

L'invention a encore pour objet l'utilisation d'un agent antitranspirant dans une composition cosmétique, en particulier de maquillage, comprenant au moins un pigment afin de limiter, diminuer et/ou de supprimer le transfert et/ou la migration de ladite composition.

L'invention a encore pour objet l'utilisation d'un agent antitranspirant dans une composition cosmétique, en particulier de maquillage, comprenant au moins un pigment, ladite composition ne transférant pas et/ou ne migrant pas sur la peau.

L'invention a encore pour objet l'utilisation d'un agent antitranspirant dans une composition cosmétique, en particulier de maquillage, à appliquer sur la peau et/ou les muqueuses et/ou le cuir chevelu, comprenant au moins un pigment, afin de diminuer la quantité d'eau présente sur la peau et/ou les muqueuses et/ou le cuir chevelu.

Les compositions selon l'invention trouvent notamment une application particulièrement intéressante dans le domaine du maquillage de la peau, des muqueuses, des semi-muqueuses, et des phanères. On entend notamment par muqueuse, la partie interne de la paupière inférieure; parmi les semi-muqueuses, on entend plus particulièrement les lèvres du visage; par phanères, on entend les cils, sourcils, cheveux et ongles. Ainsi, l'invention trouve une application toute particulière dans le domaine des produits de maquillage du visage et de la peau, tels que les fonds de teint, les autobronzants ou les produits solaires.

Un autre objet de l'invention est un procédé de traitement non thérapeutique de la peau et/ou des muqueuses et/ou du cuir chevelu, notamment un procédé de maquillage, consistant à appliquer sur la peau et/ou les muqueuses et/ou sur le cuir chevelu une composition telle que définie ci-dessus.

Certes, il est connu d'utiliser des agents antitranspirants dans des compositions telles que les déodorants. Toutefois, personne n'a pensé jusqu'à ce jour à utiliser ces agents pour des compositions de maquillage sur le visage en raison de problèmes potentiels d'inconfort (irritation, tiraillement, etc...).

Les compositions selon l'invention comprennent une phase pulvérulente qui comprend au moins un pigment. Par pigments, il faut comprendre des particules blanches ou colorées, minérales ou organiques, insolubles dans le milieu, destinées à colorer et/ou opacifier la composition.

Les pigments peuvent être présents à raison de 0,1-30 % en poids, par rapport au poids total de la composition, et de préférence à raison de 2-15 %. Ils peuvent être blancs ou colorés, minéraux et/ou organiques, de taille usuelle ou nanométrique.

On peut citer, parmi les pigments et les nanopigments minéraux, les dioxydes de titane, de zirconium ou de cérium, ainsi que les oxydes de zinc, de fer ou de chrome, les nanotitanes, le bleu ferrique.

Parmi les pigments organiques, on peut citer le noir de carbone, et les laques couramment employées pour conférer aux lèvres et à la peau un effet de maquillage, qui sont des sels de calcium, de baryum, d'aluminium ou de zirconium, de colorants acides tels que les colorants halogéno-acides, azoïques ou anthraquinoniques.

Ces pigments peuvent être traités de manière à rendre leur surface hydrophobe; ce traitement peut être effectué selon les méthodes connues de l'homme du métier; les pigments peuvent notamment être enrobés par des composés siliconés tels que des PDMS et/ou par des polymères.

La phase pulvérulente des compositions selon l'invention peut comprendre, outre les pigments cités ci-dessus, des nacres et/ou des charges habituellement utilisés dans les compositions cosmétiques. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage. Par nacres, il faut comprendre des particules irisées qui réfléchissent la lumière.

Les nacres peuvent être présentes dans la composition à raison de 0-20 % en poids, de préférence à un taux élevé de l'ordre de 2-15 % en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

Les charges, qui peuvent être présentes dans la composition à raison de 0-20 % en poids, par rapport au poids total de la composition, de préférence 2-10 %, peuvent être minérales ou de synthèse, lamellaires ou non lamellaires. On peut citer le talc, le mica, la silice, le kaolin, les poudres de Nylon et de polyéthylène, le Téflon, l'amidon, le nitrure de bore, les microsphères telles que l'Expancel (Nobel Industrie), les microéponges comme le polytrap (Dow Corning) et les microbilles de résine de silicone (Tospearls de Toshiba, par exemple).

La phase pulvérulente des compositions selon l'invention est présente à une teneur au plus égale à 80 % en poids, par rapport au poids total de la composition.

De préférence, les compositions selon l'invention ne sont pas transparentes, c'est-à-dire qu'on ne peut pas voir les caractères d'une page de journal à travers la composition. De préférence encore, elles sont colorées et de préférence encore pigmentées.

La composition selon l'invention comprend en outre un agent antitranspirant. Par agent antitranspirant, il faut comprendre un agent limitant la production de la sueur délivré sur la peau. Cet agent antitranspirant n'a pas de fonction colorante. Cet agent peut se présenter sous la forme d'un composé pulvérulent, d'une solution ou d'une dispersion.

Comme agent antitranspirant convenant particulièrement aux compositions selon l'invention, on peut citer :
- les sels d'aluminium tels que l'aluminium chlorohydrate ([Al₂(OH)₅Cl]nH₂O), l'aluminium sesquichlorohydrate ([Al₂(OH)_{4,5}Cl_{1,5}]nH₂O), l'aluminium dichlorohydrate ([Al₂(OH)₄Cl₂]nH₂O, l'aluminium chlorohydrex propylène glycol (PG) ou polyéthylène glycol (PEG) ([Al₂(OH)₅Cl]nH₂O + CH₃CHOHCH₂OH ou H(OCH₂(CH₂)nOH)), l'aluminium sesquichlorohydrex PG ou PEG, l'aluminium PG ou PEG dichlorohydrex, l'aluminium hydroxide (Al(OH)₃nH₂O),
- les chlorhydrates d'aluminium zirconium tels que l'aluminium zirconium trichlorohydrate ([Al_{3,8}Zr(OH)_{12,4}Cl₃]nH₂O), l'aluminium zirconium tétrachlorohydrate ([Al_{3,6}Zr(OH)_{11,6}Cl_{3,2}]nH₂O), l'aluminium zirconium pentachlorohydrate ([Al₈Zr(OH)₂₃Cl₅]nH₂O), l'aluminium zirconium octachlorohydrate ([Al₈Zr(OH)₂₀Cl₈]nH₂O, l'aluminium zirconium trichlorohydrex glycine ([Al_{3,8}Zr(OH)_{12,4}Cl₃]nH₂O + H₂NCH₂COOH), l'aluminium zirconium tétrachlorohydrex glycine, l'aluminium zirconium pentachlorohydrex glycine, l'aluminium zirconium octachlorohydrex glycine,
- le sulfate d'aluminium et de potassium encore appelé alun (KAl(SO₄)₂12H₂O), l'aluminium undecylenoyl collagen aminoacide, le sodium aluminium lactate + sulfate d'aluminium (Al₂(SO₄)₃ + Na₂HAl(OOCCHOHCH₃)₂-(OH)₆), le sodium aluminium chlorhydroxylactate, l'aluminium bromohydrate (Al₂Br(OH)₅nH₂O), le chlorure d'aluminium (AlCl₃6H₂O), les complexes de sel de zinc et de sodium, les complexes de lanthanium et de cérium, le sel d'aluminium de lipoaminoacide (R-CO-NH-CHR'-CO-OAl-(OH)₂ avec R = C₆/C₁₁ et R' = acide aminé).

De préférence, l'agent antitranspirant est un sel d'aluminium et de préférence encore, il est choisi parmi le sulfate d'aluminium et de potassium (alun) et le chlorhydrate d'aluminium.

De préférence, les compositions de l'invention comprennent une quantité efficace d'agent antitranspirant permettant de limiter la migration sur la peau du maquillage après application de ce dernier.

Ainsi, l'agent antitranspirant est généralement présent dans les compositions de l'invention à une teneur allant de 0,1 à 30 %, en poids, par rapport au poids total de la composition, de préférence de 1 à 10% en poids, par rapport au poids total de la composition.

Les compositions de l'invention contiennent en outre un milieu cosmétiquement, hygiéniquement, pharmaceutiquement ou dermatologiquement acceptable, c'est-à-dire un milieu compatible avec toutes les matières kératiniques telles que la peau, les ongles, les cheveux, les cils et sourcils, les muqueuses et les semi-muqueuses, et toute autre zone cutanée du corps et du visage.

Les compositions de l'invention peuvent ainsi se présenter sous la forme d'une émulsion huile-dans-eau (H/E), une émulsion eau-dans-huile (E/H), une émulsion multiple ou une solution multiphasée. Elles peuvent également se présenter sous la forme d'un gel aqueux, d'une solution aqueuse, hydroalcoolique ou multiphasée. Elles peuvent encore se présenter sous la forme d'un gel anhydre.

Les compositions de l'invention peuvent également se présenter sous la forme d'une dispersion vésiculaire, par exemple sous la forme d'une phase huileuse dispersée dans une phase aqueuse et stabilisée par des liposomes.

Lorsque la composition se présente sous forme d'une solution ou d'un gel aqueux, elle peut comprendre en outre des colorants hydrosolubles choisis parmi les colorants usuels du domaine considéré tels que le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

Lorsque la composition se présente sous la forme d'une émulsion H/E, la teneur en agent antitranspirant peut par exemple aller de 1 à 5 % ; lorsque la composition se présente sous la forme d'une émulsion E/H, cette teneur peut aller de 1 à 10 % ; lorsque la composition se présente sous la forme d'un gel aqueux, cette teneur peut aller de 1 à 5 % ; lorsque la composition se présente sous la forme d'un gel anhydre, cette teneur peut aller de 1 à 30 %.

Cette teneur est de préférence choisie par l'homme du métier afin d'obtenir des compositions stables, quelle que soit la forme de ces compositions.

Lorsque la composition selon l'invention comprend une phase aqueuse, cette dernière peut comprendre de l'eau, une eau florale telle que l'eau de bleuet et/ou une eau minérale telle que l'eau de VITTEL, l'eau de LUCAS ou l'eau de LA ROCHE POSAY et/ou une eau thermale.

Lorsque la composition selon l'invention comprend une phase grasse, cette dernière peut notamment être constituée de corps gras liquides à 25 °C, tels que des huiles d'origine animale, végétale, minérale ou synthétique.

Lorsque la composition selon l'invention se présente sous la forme d'une émulsion, ladite phase grasse peut comprendre toute huile cosmétiquement acceptable, dans la mesure où ladite huile permet, en mélange avec la phase aqueuse et les éventuels additifs, l'obtention d'une émulsion stable, c'est-à-dire d'une émulsion qui ne casse pas, qui reste sous forme d'une phase unique pendant au moins 24 heures après stockage à 25 °C, sans phénomène de crémage ou de relarguage d'huile.

Les huiles susceptibles d'être employées peuvent éventuellement être volatiles à température ambiante (20-25 °C). On entend par huile volatile, tout composé susceptible de s'évaporer au contact de la peau. De préférence, on utilise des huiles dont le point éclair est suffisamment élevé pour permettre l'utilisation de ces huiles en formulation.

Ces composés volatils peuvent être choisis en particulier parmi les huiles hydrocarbonées et/ou les huiles siliconées, cycliques ou linéaires, seules ou en mélange. On peut ainsi citer les huiles siliconées volatiles, telles que :
- les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,
- les cyclocopolymères du type diméthylsiloxane/méthylalkylsiloxane, tels que la SILICONE FZ 3109 vendue par la société UNION CARBIDE , qui est un cyclocopolymère diméthylsiloxane/méthyloctylsiloxane,
- les silicones volatiles linéaires ayant de 2 à 9 atomes de silicium. Il s'agit par exemple de l'hexaméthyldisiloxane ou un PDMS de faible viscosité (1 cSt). On peut encore citer les alkyltrisiloxanes tels que l'hexylheptaméthyltrisiloxane ou l'octylheptaméthyltrisiloxane.

On peut également citer les huiles volatiles hydrocarbonées, telles que les isoparaffines et notamment l'isododécane.

Parmi les huiles non volatiles, on peut citer :
- les polyalkyl(C₁-C₂₀) siloxanes et notamment ceux à groupements terminaux triméthylsilyle, de préférence ceux dont la viscosité est inférieure à 0,06 m²/s parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées,
- les huiles d'origine animale, végétale ou minérale, telles que l'huile de paraffine, de vaseline, le perhydrosqualène, l'huile d'abricot, l'huile de germes de blé, d'amande douce, de calophyllum, de sésame, de macadamia, de pépins de raisin, de colza, de coprah, d'arachide, de palme, de ricin, d'avocat, de jojoba, d'olive ou de germes de céréales; des esters d'acides gras et de polyol, en particulier les triglycérides liquides; des alcools; des acétylglycérides; des octanoates, décanoates ou ricinoléates d'alcools ou de polyalcools; des triglycérides d'acides gras; des glycérides, les huiles fluorées et perfluorées;
- leurs mélanges.

La composition selon l'invention peut comprendre en outre d'autres corps gras, qui peuvent être choisis par l'homme du métier sur base de ses connaissances générales, de manière à conférer à la composition finale les propriétés souhaitées, par exemple en consistance, en texture et/ou en transfert. Ces corps gras additionnels peuvent être des cires, des gommes et/ou des corps gras pâteux d'origine animale, végétale, minérale ou synthétique, ainsi que leurs mélanges.

On peut notamment citer :
- les gommes de silicones,
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; des huiles hydrogénées concrètes à 25°C; des lanolines; des esters gras concrets à 25°C; les cires de silicone; les cires fluorées; leurs mélanges.

Lorsque la composition selon l'invention se présente sous la forme d'une émulsion, elle peut éventuellement comprendre en outre un tensioactif. Comme tensioactif H/E, on peut citer notamment (CTFA) : le cétéaryl-glucoside, le PEG-40 stéarate, le sorbitan tristéarate, le sorbitan stéarate, le polysorbate 60, le mélange sorbitan stéarate/sucrose cocoate, le mélange de glycéryl stéarate/PEG-100 stéarate, le PEG-400, le stéarate de glycéryle, le mélange de PEG-6/PEG-32/glycol stéarate, le stéarate de triéthanolamine, le stéarate de sodium. Comme tensioactif E/H, on peut citer notamment le mélange polyglycéryl-4 isostéarate/ cétyldiméthicone copolyol/ hexyl laurate, le mélange Mineral oil/petrolatum/ozokérite/glycéryl oléate/alcool de lanoline, les diméthicone copolyols, les cétyl diméthicone copolyols, les diméthicone copolyols substitués en α-ω, c'est-à-dire aux deux extrémités de la chaîne siliconée. De préférence, on utilise les diméthicone copolyols substitués en α-ω.

Dans une forme préférée de réalisation, la composition selon l'invention se présente sous la forme d'une émulsion E/H.

La composition selon l'invention peut également comprendre 0 à 5 % en poids, par rapport au poids total de l'émulsion, d'au moins un co-émulsionnant qui peut être choisi parmi le monostéarate de sorbitan oxyéthyléné, des alcools gras tels que l'alcool stéarylique ou l'alcool cétylique, ou des esters d'acides gras et de polyols tels que le stéarate de glycéryle ou le polyglycéryl 10-décaoléate.

En outre, la composition selon l'invention peut comprendre un ou plusieurs agents épaississants.

La composition peut comprendre en outre tout composé complémentaire usuellement utilisé dans le domaine cosmétique, tel que des antioxydants, des parfums, des huiles essentielles, des conservateurs, des actifs cosmétiques ou pharmaceutiques lipophiles ou hydrophiles, des hydratants, des vitamines, des acides gras essentiels, des sphingolipides, des composés auto-bronzants tels que la DHA, des filtres solaires.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Ces composés complémentaires peuvent être présents dans la composition à raison de 0-10 % en poids. Selon leur nature, ils sont présents dans la phase aqueuse ou dans la phase grasse de la composition.

Les compositions selon l'invention peuvent se présenter sous toutes les formes appropriées pour une application topique, notamment sous forme de stick, de lotion, de crème, de lait, de gel aqueux ou anhydre, d'émulsions obtenues par dispersion d'une phase grasse dans une phase aqueuse (H/E) ou inversement (E/H), de consistance liquide ou semi-liquide, voire pâteuse ou solide.

Les compositions selon l'invention peuvent se présenter sous la forme d'un produit cosmétique de maquillage, notamment de la peau et/ou des muqueuses et/ou le cuir chevelu, en particulier pour le corps et/ou le visage, comme par exemple un fond de teint, un fard à joues ou à paupières, un eye-liner, un mascara ou un rouge à lèvres.

Les compositions selon l'invention peuvent constituer tout ou partie d'une composition cosmétique, pharmaceutique ou hygiénique.

Les compositions selon l'invention peuvent être préparées selon les méthodes de préparation classiques des émulsions E/H , H/E, des gels aqueux ou anhydres, ces méthodes étant bien connues de l'homme du métier.

L'invention est illustrée plus en détails dans les exemples suivants. Dans tous ces exemples, les quantités sont exprimées en pourcentage de poids par rapport au poids total de la composition.

### EXEMPLE 1:

La Demanderesse a préparé l'émulsion E/H suivante :

### Fond de Teint :

- Mélange polyglycéryl-4-isostéarate/cétyl diméthicone copolyol/héxyl laurate vendu sous la dénomination commerciale "Abil WE 09" par GOLDSCHMIDT 5 %
- cyclopentasiloxane 20 %
- huile d'abricot 5 %
- pigments enrobés PDMS 8 %
- conservateur qs
- hydroxyde d'aluminium 10 % (agent antitranspirant)
- eau qsp 100 %

Cette composition a été préparée de la manière suivante : on prépare chacune des phases aqueuse et huileuse séparément par mélange des divers composants. On incorpore ensuite la phase aqueuse dans la phase huileuse à froid sous agitation.

Cette composition est stable dans le temps. Après application sur le visage, la répartition de la couleur reste constante, même dans un climat chaud et humide et ce après plusieurs heures.

### EXEMPLE 2 :

La Demanderesse a préparé l'émulsion E/H suivante :
- Mélange silicone oxyéthylénée, oxypropylénée substituée en α-ω/cyclométhicone (85/15) vendu sous la dénomination commerciale " Abil EM 97" par GOLDSCHMIDT 6,3 %
- isostéaryl diglycéryl succinate vendu sous la dénomination commerciale "lnwitor 780 K" par HÜLS 2 %
- cyclopentasiloxane 19,35 %
- pigments 7 %
- huile volatile cyclopentasiloxane 3,5 %
- alun (agent antitranspirant) 2 %

Cette composition a été préparée selon le même procédé de préparation que la composition de l'exemple 1.

Cette composition est stable dans le temps. Après application sur le visage, la répartition de la couleur reste constante, même dans un climat chaud et humide et ce après plusieurs heures.

### EXEMPLE 3 :

La Demanderesse a préparé l'émulsion H/E suivante :
- Acide stéarique 4,9 %
- triéthanolamine 1 %
- huiles végétales 14,15 %
- cyclopentasiloxane 5 %
- polydiméthylsiloxane 10 cst 4 %
- talc 8 %
- silicate de magnésium et d'aluminium vendu sous la dénomination commerciale "Veegum" par DE VANDERBILT 2,56 %
- carboxyméthylcellulose 0,14 %
- pigments 16 %
- hydratant 6 %
- chlorhydrate d'aluminium (agent antitranspirant) 2 %
- conservateur qs
- eau qsp 100 %

La composition a été préparée selon les méthodes classiques de préparation des émulsions H/E, bien connues de l'homme du métier.

### EXEMPLE 4 (Comparatif):

La Demanderesse a réalisé 2 émulsions eau-dans-huile A (comparative) et B (invention) telles que :

### Composition :

- Mélange silicone oxyéthylénée, oxypropylénée substituée en α-ω/cyclométhicone (85/15) vendu sous la dénomination commerciale " Abil EM 97" par GOLDSCHMIDT 6,3 %
- isostéaryl diglycéryl succinate vendu sous la dénomination commerciale "lnwitor 780 K" par HÜLS 2 %
- polydiméthylsiloxane vendu sous la dénomination commerciale "Mirasil C. DPDM" par RHODIA CHEMIE 8 %
- pigments enrobés PDMS 7 %
- cyclopentasiloxane 3,5 %
- smectite 4 %
- poudre de Nylon 8 %
- M_{g}SO₄ 0,7 %
- conservateur qs
- agent antitranspirant x %
- eau qsp 100 %

| **Composition** | **Agent antitranspirant** | **x %** |
|---|---|---|
| Composition A (Comparative) | NON | 0 |
| Composition B (Invention) | Sulfate de potassium et d'aluminium 12H₂O | 5 |

La Demanderesse a réalisé des tests d'hydratation sur un panel de sept modèles, transpirant à la chaleur, placés dans une salle à la température d'environ 30°C et d'humidité relative environ 50%. On a mesuré l'hydratation sur la peau à trois reprises, sur chaque modèle, à l'aide d'un cornéomètre SEI-M-0034-COMB-02, en essuyant la sonde du cornéomètre entre chaque mesure. Ces mesures ont été mesurées à T0 (au moment de l'application du produit), après 15 minutes (T15), après 1h30 min (T1h30) et après 3 h (T3h). Les moyennes des valeurs lues au cornéomètre sont rassemblées dans le tableau suivant:

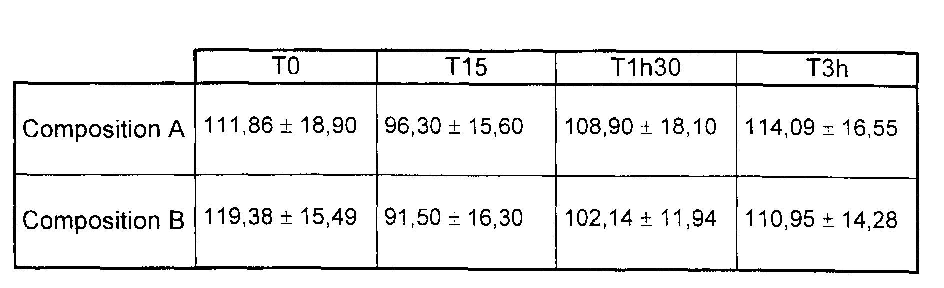

On constate que, dès 15 minutes et jusqu'à 3 heures, l'hydratation mesurée au cornéomètre est plus faible pour la composition B selon l'invention que pour la composition A non conforme à l'invention, et ce, bien qu'à T0, l'hydratation mesurée sur la composition B était supérieure. La composition B selon l'invention diminue donc la quantité d'eau présente à la surface de la peau contribuant ainsi à éviter la sensation de poisseux que l'on peut ressentir habituellement dans une atmosphère chaude et humide.

Sur le visage, la composition B migre très peu, on ne retrouve pas d'accumulation du produit dans les plis de la peau, même après plusieurs heures.

De plus, la composition B donne un teint plus mat que la composition A qui donne un teint brillant.

### EXEMPLE 5 (Comparatif):

La Demanderesse a réalisé la composition C selon l'invention correspondant à la composition B de l'exemple 4 dans laquelle les 5 % de sulfate de potassium et d'aluminium, 12H₂O ont été remplacés par 20 % de chlorhydrate d'aluminium.

La Demanderesse a ensuite évalué le transfert des compositions A et C selon le protocole suivant : une collerette en tissu a été posée sur les visages de six personnes durant 30 minutes et ce 2 heures 30 minutes après application des compositions. On a ensuite visualisé les dépôts de fond de teint sur la collerette. Les résultats sont rassemblés dans le tableau suivant:

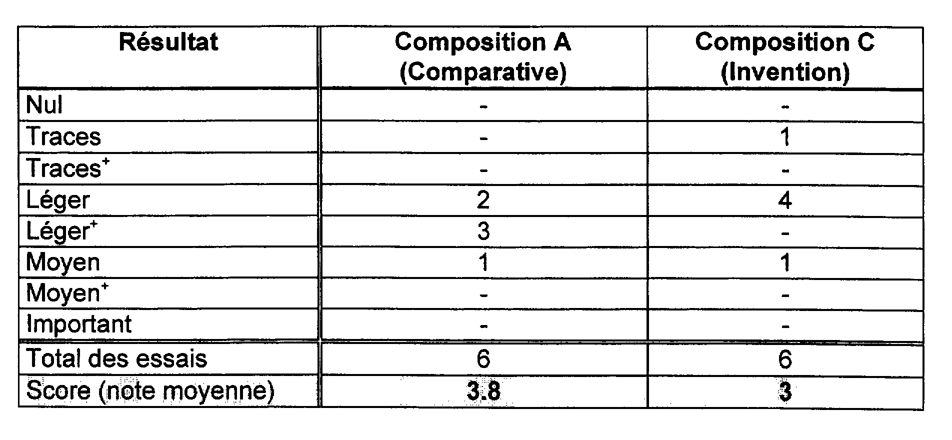

Il ressort clairement de ce tableau que la composition C selon l'invention transfère moins que la composition A qui ne comprend pas d'agent antitranspirant.

De plus, la composition C confère à la peau un teint plus mat, moins brillant que la composition A. Le film formé sur la peau par la composition C est également plus uniforme : avec la composition A, on constate au contraire la formation de plaques donnant un aspect marbré inesthétique.

## Revendications

1. Composition cosmétique, notamment de maquillage, comprenant une phase pulvérulente à une teneur inférieure ou égale à 80 % en poids, par rapport au poids total de la composition, cette phase pulvérulente comprenant au moins un pigment, caractérisée en ce qu'elle comprend en outre au moins un agent antitranspirant.

2. Composition selon la revendication 1, caractérisée en ce que le pigment est présent à une teneur allant de 0,1 à 30 % en poids, par rapport au poids total de la composition.

3. Composition selon la revendication 2, caractérisée en ce que cette teneur va de 2 à 15 %.

4. Composition selon l'une quelconque des revendications 1 à 3, caractérisée en ce que les pigments sont choisis parmi les dioxydes de titane, de zirconium ou de cérium, les oxydes de zinc, de fer ou de chrome, les nanotitanes, le bleu ferrique, le noir de carbone, les sels de calcium, de baryum, d'aluminium ou de zirconium, de colorants acides tels que les colorants halogéno-acides, azoïques ou anthraquinoniques.

5. Composition selon l'une quelconque des revendications précédentes, caractérisée en ce qu'elle n'est pas transparente.

6. Composition selon l'une quelconque des revendications précédentes, caractérisée en ce que l'agent antitranspirant est choisi parmi :
- les sels d'aluminium tels que l'aluminium chlorohydrate ([Al₂(OH)₅Cl]nH₂O), l'aluminium sesquichlorohydrate ([Al₂(OH)_{4,5}Cl_{1,5}]nH₂O), l'aluminium dichlorohydrate ([Al₂(OH)₄Cl₂]nH₂O, l'aluminium chlorohydrex propylène glycol (PG) ou polyéthylène glycol (PEG) ([Al₂(OH)₅Cl]nH₂O + CH₃CHOHCH₂OH ou H(OCH₂(CH₂)nOH)), l'aluminium sesquichlorohydrex PG ou PEG, l'aluminium PG ou PEG dichlorohydrex, l'aluminium hydroxide (Al(OH)₃nH₂O),
- les chlorhydrates d'aluminium zirconium tels que l'aluminium zirconium trichlorohydrate ([Al_{3,8}Zr(OH)_{12,4}Cl₃]nH₂O), l'aluminium zirconium tétrachlorohydrate ([Al_{3,6}Zr(OH)_{11,6}Cl_{3,2}]nH₂O), l'aluminium zirconium pentachlorohydrate ([Al₈Zr(OH)₂₃Cl₅]nH₂O), l'aluminium zirconium octachlorohydrate ([Al₈Zr(OH)₂₀Cl₈]nH₂O, l'aluminium zirconium trichlorohydrex glycine ([Al_{3,8}Zr(OH)_{12,4}Cl₃]nH₂O + H₂NCH₂COOH), l'aluminium zirconium tétrachlorohydrex glycine, l'aluminium zirconium pentachlorohydrex glycine, l'aluminium zirconium octachlorohydrex glycine,
- le sulfate d'aluminium et de potassium (KAl(SO₄)₂12H₂O), l'aluminium undecylenoyl collagen aminoacide, le sodium aluminium lactate + sulfate d'aluminium (Al₂(SO₄)₃ + Na₂HAl(OOCCHOHCH₃)₂-(OH)₆), le sodium aluminium chlorhydroxylactate, l'aluminium bromohydrate (Al₂Br(OH)₅nH₂O), le chlorure d'aluminium (AlCl₃6H₂O), les complexes de sel de zinc et de sodium, les complexes de lanthanium et de cérium, le sel d'aluminium de lipoaminoacide.

7. Composition selon la revendication 6, caractérisée en ce que l'agent antitranspirant est un sel d'aluminium.

8. Composition selon la revendication 7, caractérisée en ce que l'agent antitranspirant est choisi parmi le sulfate d'aluminium et de potassium et le chlorhydrate d'aluminium.

9. Composition selon l'une quelconque des revendications précédentes, caractérisée en ce que l'agent antitranspirant est présent dans la composition à une teneur allant de 0,1 à 30 % en poids, par rapport au poids total de la composition.

10. Composition selon l'une quelconque des revendications précédentes, caractérisée en ce qu'elle se présente sous la forme d'une émulsion E/H.

11. Composition selon l'une quelconque des revendications précédentes, caractérisée par le fait qu'elle se présente sous la forme d'un produit cosmétique de maquillage du corps et/ou du visage, en particulier d'un fond de teint, d'un fard à joues ou à paupières, d'un eye-liner, d'un mascara ou d'un rouge à lèvres.

12. Utilisation d'un agent antitranspirant dans une composition cosmétique, en particulier de maquillage, comprenant au moins un pigment afin de limiter, diminuer et/ou de supprimer le transfert et/ou la migration de ladite composition.

13. Utilisation d'un agent antitranspirant dans une composition cosmétique, en particulier de maquillage, comprenant au moins un pigment, ladite composition ne transférant pas et/ou ne migrant pas sur la peau.

14. Utilisation d'un agent antitranspirant dans une composition cosmétique, en particulier de maquillage, à appliquer sur la peau et/ou les muqueuses et/ou le cuir chevelu, comprenant au moins un pigment, afin de diminuer la quantité d'eau présente sur la peau et/ou les muqueuses et/ou le cuir chevelu.

15. Procédé de traitement non thérapeutique de la peau et/ou des muqueuses et/ou du cuir chevelu, notamment un procédé de maquillage, consistant à appliquer sur la peau et/ou les muqueuses et/ou sur le cuir chevelu une composition telle que définie à l'une quelconque des revendications 1 à 11.
